**Europäisches Patentamt**

**European Patent Office**

**Office européen des brevets**

(11) Numéro de publication : **0 389 379 B1**

(12) **FASCICULE DE BREVET EUROPEEN**

(45) Date de publication du fascicule du brevet :
**23.12.92 Bulletin 92/52**

(51) Int. Cl.⁵ : **A61F 5/02**

(21) Numéro de dépôt : **90400803.4**

(22) Date de dépôt : **23.03.90**

(54) **Orthèse pour la réduction tridimensionnelle des scolioses.**

(30) Priorité : **23.03.89 FR 8903833**

(43) Date de publication de la demande :
**26.09.90 Bulletin 90/39**

(45) Mention de la délivrance du brevet :
**23.12.92 Bulletin 92/52**

(84) Etats contractants désignés :
**BE CH DE DK ES GB IT LI NL SE**

(56) Documents cités :
**CH-A- 281 887**
**DE-C- 283 544**
**FR-A- 2 085 624**
**US-A- 4 463 752**

(73) Titulaire : **ETABLISSEMENTS PROTEOR**
**11 rue des Buttes**
**F-21100 Dijon (FR)**

(72) Inventeur : **Graf, Henry**
**8, rue Duquesne**
**F-69006 Lyon (FR)**
Inventeur : **Dauny, Gérald**
**17, rue des Murgers**
**F-21240 Savigny Les Beaune (FR)**

(74) Mandataire : **Jolly, Jean-Pierre et al**
**Cabinet Jolly 54, rue de Clichy**
**F-75009 Paris (FR)**

EP 0 389 379 B1

Jouve, 18, rue Saint-Denis, 75001 PARIS

## Description

La présente invention concerne une orthèse pour la réduction tridimensionnelle des scolioses.

On sait que la scoliose peut être analysée comme une déformation segmentaire du tronc, chaque segment comprenant une gibbosité d'un côté et une contre-vallée de l'autre côté. Chaque segment s'articule sur le segment adjacent par une ligne oblique, décrite par le Docteur CHENEAU comme étant une ligne neutre (voir publication CHENEAU-MUNSTER). Cette ligne neutre correspond, sur le plan radiologique, au noeud de jonction défini par Monsieur PERDRIOLLE et par le Docteur GRAF (voir Pedriolle : "La scoliose, son étude tridimensionnelle", Graf : "Mécanique de la courbe scoliotique").

Pour réduire de telles scolioses, on a déjà proposé divers types de corsets, comprenant des éléments en forme de plaques, que l'on appelle des "mains" dans la technique, qui sollicitent des parties du thorax ou du dos du patient pour redresser la colonne vertébrale et la ramener en position verticale, tout en exerçant sur les vertèbres un effet de dérotation et d'élongation, pour compenser le pivotement et l'affaissement des vertèbres qui se manifestent dans une scoliose. Un ensemble de telles orthèses est décrit dans la publication FR-A-2 085 624.

Les "mains" de tels corsets sont réglables en position, afin de pouvoir moduler et adapter leur action à mesure que progresse la réduction de la scoliose. Elles sont cependant purement "passives", en ce sens qu'elles sont fixes en position par rapport au corps du patient et exercent sur celui-ci une simple pression dont la direction d'appplication et la grandeur de l'effort ne sont pas modulés en fonction du cycle respiratoire.

La présente invention vise à perfectionner les corsets de ce type en proposant une orthèse qui, au cours de la phase d'inspiration du cycle respiratoire du patient, s'oppose à l'expansion naturelle de la cage thoracique par l'avant en forçant le patient à se voûter, ce qui amène le rachis dorsal en position de cyphose, pour normaliser ainsi la position vertébrale.

Plus précisément, l'invention vise à proposer une orthèse pour la réduction tridimensionnelle des scolioses, qui, à la phase d'inspiration du cycle respiratoire impose au patient de se voûter, lorsque les omoplates s'écartent l'une de l'autre, et à sa colonne vertébrale de se redresser, avec un effet inverse à l'expiration. Cet effet de mouvement permanent de l'orthèse, sous la sollicitation même du patient, entraîne une rééducation active du rachis.

Lors de leur phase d'écartement, les tuteurs imposent aux mains des mouvements de rotation-compression ayant pour but une détorsion des segments sus et sous-jacents au noeud de jonction, l'un par rapport à l'autre.

L'invention a par conséquent pour objet une orthèse pour la réduction tridimensionnelle d'une scoliose, caractérisée en ce qu'elle comprend :
- d'une part, deux mains latérales en une matière plastique déformable élastiquement, destinées à enserrer latéralement le thorax du patient, avec une partie antérieure disposée à l'avant du thorax et une partie postérieure disposée à l'arrière de celui-ci, ces mains étant solidarisées antérieurement par des moyens d'assemblage qui leur interdisent de s'écarter l'une de l'autre, tandis que les parties postérieures sont libres de se déplacer l'une par rapport à l'autre lorsqu'elles sont sollicitées par le thorax au cours du cycle respiratoire ;
- d'autre part, une ceinture pelvienne destinée à être rendue solidaire du bassin du patient ;
- enfin, au moins deux tuteurs latéraux en une matière semi-rigide déformable élastiquement, qui réunissent respectivement en direction verticale l'une desdites mains et la partie correspondante de la ceinture pelvienne, en vue d'exercer un effort de rappel en torsion sur les parties postérieures des mains lorsque celles-ci s'écartent l'une de l'autre en phase d'inspiration du thorax du patient.

Une main d'appui lombaire en matière plastique peut être ajoutée; cette main peut être active ou passive.

On conçoit que, les mains étant réunies par des moyens d'assemblage antérieurs, tandis que les parties postérieures sont libres de se déplacer élastiquement, l'orthèse impose aux mouvements respiratoires spontanés du patient de s'exercer par l'arrière, en recréant ainsi une cyphose physiologique propice à la réduction de la scoliose.

Les mains de l'orthèse pourront être en tout matériau usuel dans la technique, par exemple en polyéthylène rigide. Les moyens d'assemblage qui les réunissent antérieurement pourront être de simples sangles. De préférence, ces moyens de réglages seront réglables en position, afin de pouvoir rendre les mains solidaires l'une de l'autre en une pluralité de positions, à mesure que progresse la rééducation du patient.

Les tuteurs pourront être avantageusement constitués de bandes d'un composite de fibres de carbone, constitué d'une résine thermoplastique armée de fibres de carbone, de préférence tissées ou tressées, et être fixés par des rivets ou similaires sur les mains et sur la ceinture pelvienne.

La ceinture pelvienne pourra être de tout type connu en soi.

Dans une forme préférée de mise en oeuvre de l'invention, l'effort de rappel en torsion exercé par les tuteurs latéraux sera réglable. Dans ce but, la partie de ces tuteurs séparant une main de la ceinture pelvienne ne pourra se déformer élastiquement en rotation que sur une partie réglable de sa longueur. A cet effet, les tuteurs seront avantageusement doublés suivant une partie de leur longueur, à partir d'une main et/ou de la ceinture pelvienne, par un élément rigide, réglable en position. Ce ou ces éléments seront par exemple montés coulissants sur les tuteurs et aptes à être rendus solidaires de ceux-ci en une pluralité de positions, par exemple à l'aide de vis ou d'écrous. Il sera ainsi possible de régler l'effort de torsion exercé en rappel par les tuteurs latéraux, lorsque les mains sont sollicitées à l'inspiration par le thorax du patient et tendent à s'écarter l'une de l'autre. Autrement dit, il sera possible de régler l'effort que devra exercer le patient, lors d'une inpiration naturelle, pour écarter l'une de l'autre les parties postérieures des mains.

Ces tuteurs peuvent avoir un réglage en toutes positions au niveau de la jonction avec la ceinture pelvienne.

L'invention propose donc une orthèse simple pour réduire tridimensionnellement une scoliose, avec laquelle le patient sollicite lui-même de façon dynamique, à l'inspiration, les mains destinées à assurer sa rééducation.

Une forme de réalisation de l'invention sera décrite ci-après, à titre d'exemple non limitatif, en référence aux dessins annexés. Sur ces dessins :

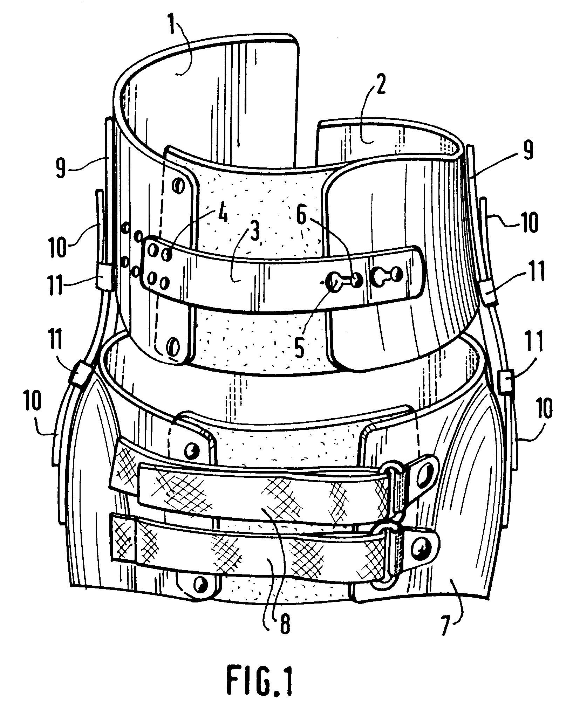

La figure 1 est une vue en perspective de face de l'orthèse conforme à l'invention ;

La figure 2 est une vue latérale de cette même orthèse ;

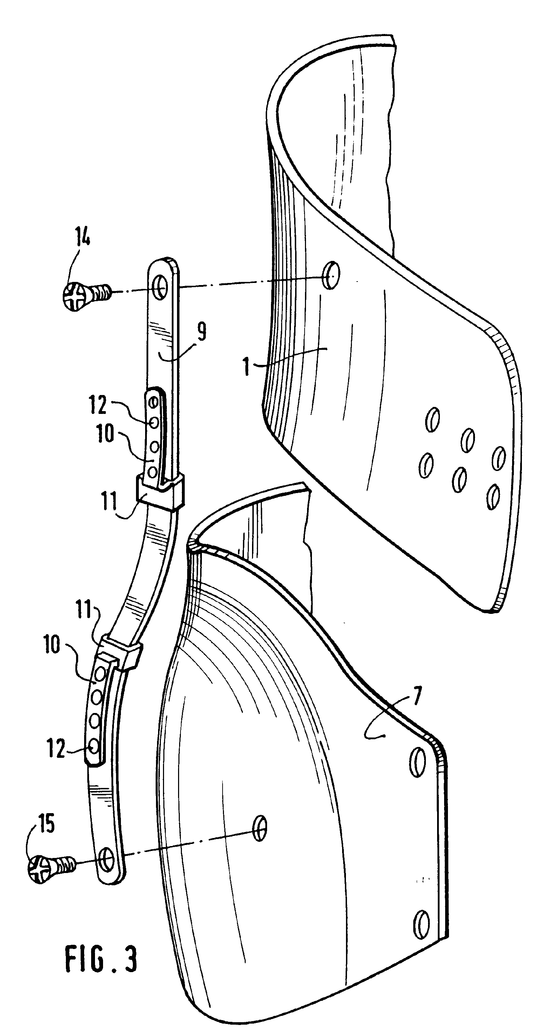

La figure 3 est une vue partielle éclatée de celle-ci.

Comme représenté sur les dessins, cette orthèse comprend deux plaques incurvées 1, 2 en matière plastique, dites "mains", qui épousent latéralement la forme du thorax du patient et dont les parties antérieures sont rendues solidaires l'une de l'autre par une sangle 3. Cette sangle est fixée à l'aide de vis 4 sur la mains 1, et son autre extrémité comprend des ouvertures 5, dans lesquelles peuvent être engagés de façon réglable un ou des boutons vissés 6 faisant saillie sur la face externe de la main. Les parties postérieures des mains 1 et 2 sont montées libres de se déplacer et, en particulier, de s'écarter l'une de l'autre.

L'orthèse comprend également une ceinture pelvienne, d'un type connu en soi, comprenant une coquille ouverte 7, dont les extrémités antérieures sont réunies de façon réglable par des sangles 8.

Des tuteurs 9 en un matériau déformable élastiquement, par exemple en composite de fibres de carbone, sont disposés verticalement pour réunir les parties latérales des mains 1 et 2 et la ceinture pelvienne 7, sur lesquelles ils sont fixés respectivement par des vis 14 et 15.

Ainsi qu'il a été expliqué ci-dessus, lors des mouvements d'inspiration du patient, la sangle 3 s'oppose à l'ouverture vers l'avant des mains 1 et 2, malgré l'augmentation de volume de la cage thoracique, ce qui contraint le patient à se voûter en écartant l'une de l'autre les parties arrières des mains 1 et 2, en amenant ainsi le rachis dorsal en position de cyphose en vue de normaliser la position de la colonne vertébrale.

Au cours du mouvement d'inspiration du patient, l'ouverture des mains 1 et 2 imprimera donc une rotation aux tuteurs 9 et le patient devra lutter contre l'effort de rappel exercé par ces tuteurs.

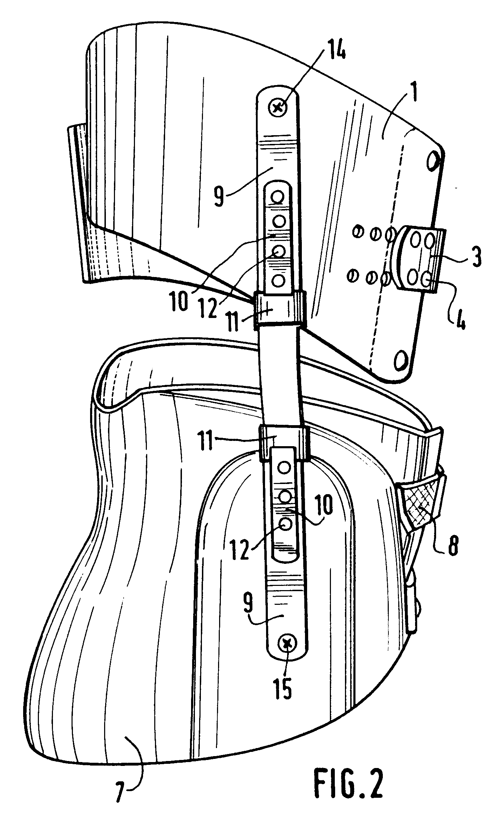

Afin de pouvoir régler cet effort, la partie des tuteurs 9 susceptible de pivoter sur elle-même sera limitée et réglable. Dans ce but, comme on le voit plus clairement sur la figure 2, chaque tuteur 9 sera doublé sur une partie de sa longueur, tant à partir des mains 1 et 2 qu'à partir de la ceinture 7, par des bandes rigides 10, montées coulissantes sur les tuteurs 9 à l'aide de passants 11 et susceptibles d'être immobilisées sur ces tuteurs en une pluralité de positions, en engageant des vis 12 dans des ouvertures des bandes 10. La seule partie des tuteurs 9 susceptible de se déformer, lorsqu'elle est soumise à un couple de torsion, est ainsi la longueur séparant les passants 11 et, plus ces passants sont proches de l'autre, plus élevé est l'effort que le patient doit exercer à l'inspiration pour faire pivoter les tuteurs et provoquer l'écartement des parties postérieures des mains 1 et 2.

L'invention apporte donc un moyen simple et réglable pour réduire tridimensionnellement de façon dynamique les scolioses.

## Revendications

1. Orthèse pour la réduction tridimensionnelle de façon dynamique d'une scoliose, comprenant :

- d'une part, deux mains latérales (1,2) en une matière plastique déformable élastiquement, destinées à enserrer latéralement le thorax du patient, avec une partie antérieure disposée à l'avant du thorax et une partie postérieure disposée à l'arrière de celui-ci, les parties antérieures de ces mains étant solidarisées par des moyens d'assemblage (3) qui leur interdisent de s'écarter l'une de l'autre, tandis que les parties postérieures sont libres de se déplacer l'une par rapport à l'autre lorsqu'elles sont sollicitées par le thorax au cours du cycle respiratoire ;

- d'autre part, une ceinture pelvienne (7) destinée à être rendue solidaire du bassin du patient ;

- enfin, au moins deux tuteurs latéraux (9) en une matière semi-rigide déformable élastiquement, qui réunissent respectivement en direction verticale l'une desdites mains (1,2) et la partie correspondante de la ceinture pelvienne (7), en vue d'exercer un effort de rappel en torsion sur les parties postérieures des mains (1,2) lorsque celles-ci s'écartent l'une de l'autre en phase d'inspiration du thorax du patient.

2. Orthèse selon la revendication 1, caractérisée en ce que le moyen d'assemblage des parties antérieures des mains (1,2) est réglable en position.

3. Orthèse selon l'une des revendications 1 et 2, caractérisée en ce que le moyen d'assemblage des parties antérieures des mains (1,2) comprend une sangle.

4. Orthèse selon l'une des revendications 1 à 3, caractérisée en ce que les tuteurs (9) sont équipés d'un moyen de réglage de leur effort de rappel en torsion.

5. Orthèse selon la revendication 4, caractérisée en ce que le moyen de réglage de l'effort de rappel en torsion des tuteurs (9) limite la longueur de ceux-ci susceptible d'être soumise à cet effet de torsion.

6. Orthèse selon la revendication 5, caractérisée en ce que le moyen de réglage de l'effort de rappel en torsion des tuteurs comprend au moins un élément rigide (10) doublant chaque tuteur (9) sur une partie de sa longueur à partir d'une main (1,2) et/ou de la ceinture pelvienne (7), cet élément rigide étant monté coulissant par rapport au tuteur (9) et susceptible d'être fixé en une pluralité de positions sur celui-ci.

7. Orthèse selon la revendication 6, caractérisée en ce que les tuteurs (9) comportent un moyen de réglage antéro-postérieur s'articulant à leur base au niveau de la ceinture pelvienne.

**Claims**

1. An orthesis for dynamic three-dimensional reduction of scoliosis, comprising:
   - on the one hand, two lateral hands (1, 2) of an elastically deformable plastics material, for lateral grasping of the thorax of the patient, with a front portion arranged at the front of the thorax and a rear portion arranged at the back thereof, the front portions of these hands being firmly connected by assembly means (3) which prevent them from separating from each other, while the rear portions are free to move with respect to each other when they are stressed by the thorax during the respiratory cycle;
   - on the other hand, a pelvic belt (7) to be fixed firmly to the pelvis of the patient;
   - finally, at least two lateral supports (9) of a semi-rigid, elastically deformable material which respectively connect vertically one of said hands (1, 2) and the corresponding part of the pelvic belt (7), with a view to exerting a torsional return force on the rear parts of the hands (1, 2) when these separate from each other in the inspiration phase of the patient's thorax.

2. An orthesis according to claim 1, characterized in that the position of the means of assembling the front portions of the hands (1, 2) can be regulated.

3. An orthesis according to either one of claims 1 and 2, characterized in that the means of assembling the front parts of the hands (1, 2) comprises a strap.

4. An orthesis according to any one of claims 1 to 3, characterized in that the supports (9) are provided with a means of regulating their torsional return force.

5. An orthesis according to claim 4, characterized in that the means of regulating the torsional return force of the supports (9) limits the length thereof capable of being subjected to this torsional force.

6. An orthesis according to claim 5, characterized in that the means of regulating the torsional return force of the supports comprises at least one rigid member (10) overlapping each;support (9) over part of its length starting from one hand (1, 2) and/or the pelvic belt (7), this rigid member being mounted slidingly with respect to the support (9) and capable of being fixed in a plurality of positions thereon.

7. An orthesis according to claim 6, characterized in that the supports (9) comprise means of front/rear regulation articulated at their basex at the level of the pelvic belt.

**Patentansprüche**

1. Orthese zur dreidimensionalen dynamischen Rückbildung einer Skoliose, welche folgendes aufweist:
   - einerseits zwei seitliche Thoraxumschließungen (1, 2) aus elastisch verformbaren Kunstoff, die zur seitlichen Umschließung des Thorax des Patienten vorgesehen sind und ein vor dem Brustkorb liegendes Vorderteil und ein dahinter liegendes Rückenteil aufweisen, wobei diese Thoraxumschließungen vorn durch Verbindungsmittel (3) fest miteinander verbunden sind, welche ein Aufspreizen der Thoraxumschließungen verhindern, während die Rückenteile frei gegeneinander beweglich sind, wenn sie während der Atmung beansprucht werden;
- andererseits einen Beckengürtel (7) zur festen Verbindung mit dem Becken des Patienten;
- schließlich mindestens zwei elastisch verformbare halbsteife seitliche Stützstäbe (9), die jeweils in senkrechter Richtung eine der Thoraxumschließungen (1, 2) mit dem entsprechenden Teil des Beckengürtels (7) so verbinden, daß auf die rückwärtigen Teile der Thoraxumschließungen (1, 2) eine Rückstellwirkung unter Torsion ausgeübt wird, wenn sich diese bei der Einatmung in den Brustraum des Patienten auseinander spreizen.

2. Orthese nach Anspruch 1, dadurch gekennzeichnet, daß die Verbindungsmittel zur Verbindung der Vorderteile der Thoraxumschließungen (1, 2) in ihrer Position einstellbar sind.

3. Orthese nach einem der Ansprüche 1 und 2, dadurch gekennzeichnet, daß die Verbindungsmittel zur Verbindung der Vorderteile der Thoraxumschließungen (1, 2) einen Gurt aufweisen.

4. Orthese nach einem der Ansprüche 1 bis 3, dadurch gekennzeichnet, daß die Stützstäbe (9) mit Mitteln zur Einstellung ihrer Rückstellkraft unter Torsion ausgerüstet sind.

5. Orthese nach Anspruch 4, dadurch gekennzeichnet, daß die Mittel zum Regulieren der Rückstellkraft der Stützstäbe (9) unter Torsion die Länge des Abschnitts der Stäbe begrenzen, der mit dieser Torsionskraft beaufschlagbar ist.

6. Orthese nach Anspruch 5, dadurch gekennzeichnet, daß die Mittel zum Regulieren der Rückstellkraft der Stützstäbe unter Torsion mindestens ein unelastisches Element (10) aufweisen, welches auf jeden Stützstab (9) über einen Teil von dessen Länge, ausgehend von einer Thoraxumschließung (1, 2) und/oder dem Beckengürtel (7), aufgelegt ist sowie gegenüber dem Stützstab (9) verschieblich angeordnet und auf diesem in mehreren Positionen fixierbar ist.

7. Orthese nach Anspruch 6, dadurch gekennzeichnet, daß die Stützstäbe (9) eine an ihrer Basis in Höhe des Beckengürtels angelenkte Einrichtung zur vorderen und hinteren Regulierung aufweisen.

FIG.1

FIG.2

FIG.3